# EUROPEAN PATENT APPLICATION

(11) **EP 4 477 234 A1**
(43) Date of publication of application: **18.12.2024**
(21) Application number: 22926037.7
(22) Date of filing: 07.10.2022
(51) Int. Cl.: A61K 45/00, A61K 31/365, A61K 39/395, A61P 35/00, A61P 43/00, A23L 33/10

(54) **CANCER-CELL PROLIFERATION INHIBITOR AND CANCER-CELL-PROLIFERATION INHIBITION ENHANCER**

(30) Priority: 09.02.2022 JP 2022018618
(71) Applicant: Kyushu University, National University Corporation, Nishi-ku Fukuoka-shi Fukuoka 819-0395 (JP); MITSUI NORIN CO., LTD., Tokyo 105-8427 (JP)
(72) Inventor: TACHIBANA Hirofumi, Fukuoka-shi, Fukuoka 819-0395 (JP); TAKAGAKI Akiko, Fujieda-shi, Shizuoka 426-0133 (JP)
(74) Representative: Clarenbach, Carl-Philipp
(86) International application number: PCT/JP2022/037606
(87) International publication number: WO 2023/153020

(57) **Abstract**

The aim of the present invention is to provide a cancer-cell proliferation inhibitor that contains a compound with a high level of safety derived from food component. The description of the present application discloses a cancer-cell proliferation inhibitor that contains an immune checkpoint inhibitor and at least one of 5-(3,5-dihydroxyphenyl)-γ-valerolactone represented by formula (I), a salt of said compound, and a conjugate of said compound.

## Description

### Technical Field

The method in which the capability of the immune cell to attack is enhanced is central to the immunotherapy thus far. Recently it has become clear that the attack of the immune cells can be inhibited by binding of the immune checkpoint on the surface of the cancer-cell to the receptor of the immune cells. The development of the method in which the capability of the immune cells to attack the cancer-cell is kept by using the immune checkpoint inhibitor is drawing attention.

The immune checkpoint inhibitor indirectly attacks the cancer-cell by blocking the binding of the immune checkpoint to the receptors and activating the immune cells that attack the cancer-cell unlike the conventional anticancer agent directly attacking the cancer-cell. Thus, the strain on the body is less, and treatment effects can sometimes be seen even in advanced cancer where anticancer drugs have become ineffective. Moreover, the immune checkpoint inhibitor activates one's own immunity, there tends to be a sustained therapeutic effect, it has relatively fewer side effect and higher safety compared to conventional anticancer drugs, which is a feature of it (Non-Patent Document 1). Therefore, the cancer treatment by immunotherapy is attracting attention as the fourth treatment method following surgery, chemotherapy, and radiotherapy, and it is a field in which further expansion is anticipated.

The respective immune checkpoint inhibitors include anti-PD-1 antibody, anti-PDL-1 antibody, and anti-CTLA-4 antibody, in particular, the use of anti-PD-1 is quite common.

On the other hand, the cancer response rate is reported to be 20 % when the immune checkpoint inhibitor is used alone, indicating that not all patients can expect therapeutic effects. Thus, improvement in the cancer response rate is anticipated from combined treatments with other therapies, and the combination with approaches such as vaccine therapy is being considered (see Patent Document 1, Non-Patent Document 2)

By the way, with respect to the immune activation properties of the metabolites of the green tea catechin, known effects include enhancing the immune cell proliferation (Patent Document 2) and activating natural killer cells (Patent Document 3). Regarding the anti-cancer action of the metabolites of the green tea catechin, the actions inhibit the proliferation of cervical cancer-cell is known (Patent Document 4), and it is also described that the combined use with flavan-3-ols enhances the effect of inhibiting cancer cell proliferation (Patent Document 5). However, the anti-cancer action of the metabolites of the green tea catechin when used alone are limited.

### Citation List

### Patent Document

Patent Document 1: WO 2020/067085 A
Patent Document 2: JP 2016-003200 A
Patent Document 3: JP 2016-160238 A
Patent Document 4: JP 2015-030724 A
Patent Document 5: JP 2015-209418 A

Non-Patent Document 1: Lancet, 2017, vol.390, p.2461-2471
Non-Patent Document 2: OncoImmunology. 2020, vol.9, p.1734268

### Summary of Invention

### Technical Problem

The aim of the present invention is to provide a cancer-cell proliferation inhibitor that contains a component with a high level of safety derived from food component. This cancer cell proliferation inhibitor has a greater anti-cancer effect than that of the immune checkpoint inhibitor used alone, and can enhance the cancer-cell proliferation inhibitory effectiveness of the immune checkpoint inhibitor.

### Solution to Problem

The present inventors examined the physiological effects of 5-(3,5-dihydroxyphenyl)-γ-valerolactone, which is reported as a major metabolite of catechins. As a result, the inventors discovered that this metabolite of catechin had the ability to enhance the anticancer effectiveness of the immune checkpoint inhibitor, leading to the completion of this invention.

That is, the present invention relates to:
[1] A cancer-cell proliferation inhibitor comprising an immune checkpoint inhibitor and at least one of 5-(3,5-dihydroxyphenyl)-γ-valerolactone, which is represented by a following formula (I), a salt of said compound, and a conjugate of said compound. 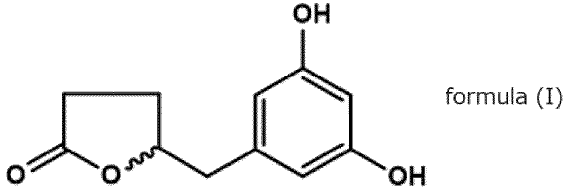
[2] The cancer-cell proliferation inhibitor according to [1], wherein the immune checkpoint inhibitor is one or more selected from a group consisting of anti-PD-1 antibody, anti-PD-L1 antibody, anti-CTLA-4 antibody, anti-B7 antibody, anti-C27 antibody, anti-KIR antibody, IDO inhibitor, anti-CD137 antibody, and anti-TIM3 antibody.
[3] The cancer-cell proliferation inhibitor according to [2], wherein the immune checkpoint inhibitor is anti-PD-1 antibody or anti-CTLA-4 antibody.
[4] A medicine for inhibiting cancer-cell proliferation containing the cancer-cell proliferation inhibitor according to any one of [1] to [3].
[5] A supplement for inhibiting cancer-cell proliferation containing the cancer-cell proliferation inhibitor according to any one of [1] to [3] .
[6] A method for enhancing effect of an immune checkpoint inhibitor, wherein anticancer effect of the immune checkpoint inhibitor is enhanced by using the immune checkpoint inhibitor and a cancer-cell proliferation inhibitor comprising at least one of 5-(3,5-dihydroxyphenyl)-γ-valerolactone, which is represented by a following formula (I), a salt of said compound, and a conjugate of said compound as an active ingredient together. 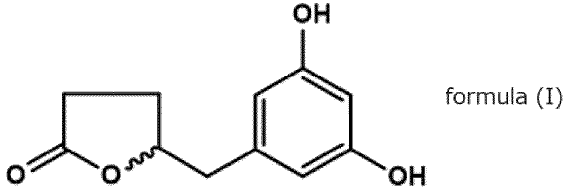
[7] A cancer-cell-proliferation inhibition effect enhancer for enhancing cancer-cell-proliferation inhibiting effect of an immune checkpoint inhibitor, comprising at least one of 5-(3,5-dihydroxyphenyl)-γ-valerolactone, which is represented by a following formula (I), a salt of said compound, and a conjugate of said compound.

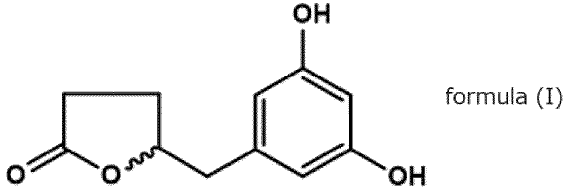

### Effects of the Invention

The present invention pertains to a cancer-cell proliferation inhibitor, which comprises an immune checkpoint inhibitor and at least one of the following: 5-(3,5-dihydroxyphenyl)-γ-valerolactone, a salt of said compound, or a conjugate of said compound. 5-(3,5-dihydroxyphenyl)-γ-valerolactone is highly safe, with its active ingredient derived from food components, yielding an immediate effect. This invention is safe, highly versatile and can be broadly applied to supplements, medicine and similar products. With this invention, we can provide functional foods, supplements, and medicine that are high in safety and efficacy in inhibiting the proliferation of cancer cells. 5-(3,5-dihydroxyphenyl)-γ-valerolactone, its salts, or conjugates can enhance the anticancer effect of the immune checkpoint inhibitor when used in combination.

### Brief Description of Drawings

[Fig.1] Fig.1 shows the tumor volume on the 20th day after transplantation of the MC38 mouse colon cancer cell line, in Example 1.
[Fig.2] Fig.2 shows the tumor volume on the 24th day after transplantation of the MC38 mouse colon cancer cell line, in Example 2.
[Fig.3] Fig.3 shows the changes in survival rate of mice from the 4th day to the 38th day after transplantation of the MC38 mouse colon cancer cell line, from Example 2.
[Fig.4] Fig.4 shows the tumor volume on the 22nd day after transplantation of the AB1 mouse malignant pleural mesothelioma cell line, as performed in Example 3.
[Fig.5] Fig.5 shows the tumor volume on the 16th day after transplantation of the MC38 mouse colon cancer cell line, from the Comparative Example.

### Form to carry out invention

The present invention is now described in more detail as follow and is not limit to the description below. The appropriate variations should be possible within the scope of the present invention.

One of the present inventions is the cancer-cell proliferation inhibitor that contains an immune checkpoint inhibitor and at least one of 5-(3,5-dihydroxyphenyl)-γ-valerolactone represented by the following formula (I), a salt of said compound, and a conjugate of said compound.

The compound contained in the cancer-cell proliferation inhibitor of the present invention is 5-(3,5-dihydroxyphenyl)-γ-valerolactone represented by the formula (I), a salt of said compound, or a conjugate of said compound.

The compound represented by the formula (I) is described. 5-(3,5-dihydroxyphenyl)-γ-valerolactone represented by the formula (I) is as follows.

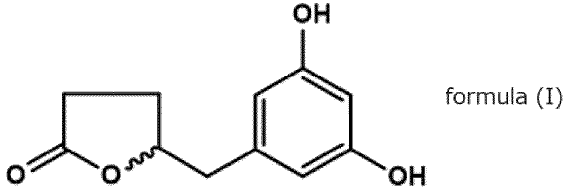

In the formula (I), the wavy line represents that the spatial configuration can be either R or S. Therefore, the compound represented by formula (I) includes both of R and S form, with the R form being preferred. This compound is recognized as a metabolite produced in the intestine when epigallocatechin or epigallocatechin gallate, types of tea catechins, are consumed orally. The manufacturing method of the compound is well-known. For example, Patent Document JP2011-87486 A discloses the production method in which the compound is produced by microorganism from epigallocatechin or the production method in which the compound is produced in the presence of the cultured microorganism preparation. In addition, the compound can be obtained by the well-known method for organic chemical synthesis (synthesis, 9, 1512-1520, 2010) and the like.

5-(3,5-dihydroxyphenyl)-γ-valerolactone is a metabolite produced by intestinal bacteria from epigallocatechin, epigallocatechin gallate, gallocatechin, or gallocatechin gallate. It is reported that the sulfate conjugate and the glucuronic acid conjugate of the compound of 5-(3,5-dihydroxyphenyl)-γ-valerolactone are detected in the serum of the mouse when orally administering 5-(3,5-dihydroxyphenyl)-γ-valerolactone to the mouse (Biol. Pharm. Bull, 42, 212-221, 2019). Accordingly, it is already known that the conjugates are absorbed into the body of the mouse after orally administering 5-(3,5-dihydroxyphenyl)-γ-valerolactone, which suggests the possibility that the conjugate contribute the biofunctional property after orally administering 5-(3,5-dihydroxyphenyl)-γ-valerolactone. Hence, the anticancer effect in the living body described in this specification can be regarded to include the effect of the conjugates of 5-(3,5-dihydroxyphenyl)-γ-valerolactone such as the sulfate conjugate and/or the glucuronic acid conjugate.

Namely, the active ingredient of the cancer-cell proliferation inhibitor of the present invention includes 5-(3,5-dihydroxyphenyl)-γ-valerolactone represented by the formula (I), a salt of said compound, and a conjugate of said compound.

Namely, the sulfate conjugate of 5-(3,5-dihydroxyphenyl)-γ-valerolactone is the compound obtained by substituting at least one hydroxy group in the compound represented by the formula (I) for sulfate group or salt of the group. The sulfate group or the salt of the group is either of the substituents described below.

-OSO₃H

-OSO₃⁻

An example of a compound containing at least one sulfate group or its salt as a substituent can be obtained by sulfonating at least one hydroxy group. The sulfonation can be carried out by using sulfur trioxide pyridine and the like.

The glucuronic acid conjugate of 5-(3,5-dihydroxyphenyl)-γ-valerolactone is the compound which is obtained by substituting at least one hydroxy group in the compound represented by the formula (I) for the substituent containing glucuronic acid or the salt of glucuronic acid. The substituent containing glucuronic acid or the salt of glucuronic acid is either of the substituents described below.

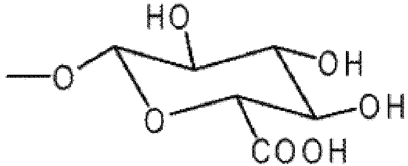

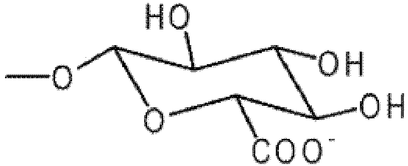

Examples of compound containing glucuronic acid or its salt in the formula (I) are metabolites of the tea catechins, which are detected in urine when ingesting substances like epigallocatechin orally. The methods for manufacturing these include the one in which the metabolites in the urine collected from a rat administered with epigallocatechin is separated and collected using HPLC and the like.

By administering the compound represented by the formula (I) in combination with the immune checkpoint inhibitor, the effect of the immune checkpoint inhibitor can be enhanced, the tumor volume can be reduced and the effect of inhibiting the cell proliferation can be sufficiently exhibited.

The cancer-cell proliferation inhibitor of the present invention can contain the salt of the compound represented by the formula (I) also having a similar cancer-cell proliferation inhibiting effect as the active ingredient. The salt of the compound represented by the formula (I) is preferably the pharmacologically permissible salt. Examples of these salt include alkali metal salt such as sodium salt and potassium salt, alkaline earth metal salt such as calcium salt and magnesium salt, ammonium salt, and the salt of organic base such as methylamine, ethylamine, butylamine, dimethylamine, diethylamine, triethylamine, tributylamine, ethanolamine, pyridine, lysine, and arginine.

These salts can be obtained by the conventional method, for example, sodium salt can be obtained by bringing the compound represented by the formula (I) into contact with sodium hydroxide.

As described above, the compound represented by the formula (I) can be obtained from epigallocatechin or is the metabolite of epigallocatechin, epigallocatechin gallate, or gallocatechin, gallocatechin gallate or the derivative of the metabolite. Catechins [epigallocatechin ((-)-epigallocatechin), epigallocatechin gallate ((-)-epigallocatechin gallate), gallocatechin ((-)-gallocatechin), gallocatechin gallate ((-)-gallocatechin gallate)] are mainly contained in the green tea and obtained by extracting the leaves, the stem, the xylem, the bark, the root, the fruit, the seed of the tea and the mixture of them or the pulverized product of them with water, hot water, organic solvent, water-containing organic solvent or the mixture of them. Besides the extract itself there is the purer form of the extract and the like. The form of catechins may be either of liquid or solid (including powder).

The cancer on which the cancer-cell proliferation inhibitor of the present invention have the effect is not particularly limited but include acute myelocytic leukemia, chronic myelogenous leukemia, malignant lymphoma, multiple myeloma, brain tumor and glioma, pituitary adenoma, acoustic neuroma, uveal melanoma, meningioma, pharyngeal cancer, laryngeal cancer, tongue cancer, thyroid cancer, breast cancer, lung cancer, thymoma, thymic cancer, mesothelioma, esophageal cancer, gastric cancer, colorectal cancer, hepatocellular carcinoma, common bile duct cancer, pancreatic cancer, renal cell carcinoma, bladder cancer, prostate cancer, renal pelvis and ureteral cancer, penile cancer, testicular tumor, uterine cancer, ovarian cancer, vulvar cancer, skin cancer, malignant melanoma (skin), base cell carcinoma, prodrome of skin cancer, intraepidermal carcinoma, squamous cell carcinoma of skin, mycosis fungoides, malignant bone tumor (osteo sarcoma),soft tissue sarcoma, chondrosarcoma, malignant fibrous histiocytoma, and metastatic cancer of the aforementioned. Particularly the cancer-cell proliferation inhibitor of the present invention is effective against colonic cancer which is one of large intestine cancer and malignant pleural mesothelioma which is one of mesothelioma.

The cancer-cell proliferation inhibitor of the present invention contains the compound represented by the above formula (I) as the active ingredient. When administering the compound to the human body, the compound is administered to avoid adverse effects on the human body. In the present invention, the suitable dosage of the compound represented by the formula (I) for a human being may be determined according to factors such as weight, sex, age, or other factor appropriately. For example, when administering orally, the administering amount is preferably 10 to 500 mg per day per adult human who weighs 60 kg, more preferably 20 to 300 mg, further preferably 20 to 150 mg. For example, the compound in an amount described above is preferably divided in 1 to 3 times per day to administer.

The cancer-cell proliferation inhibitor of the present invention contains the immune checkpoint inhibitor. The immune checkpoint inhibitor has the capability of inhibiting the cancer-cell proliferation even when used alone. But by using in combination with at least one of 5-(3,5-dihydroxyphenyl)-γ-valerolactone represented by the formula (I), a salt of said compound, and a conjugate of said compound, the effect of inhibiting cancer-cell proliferation can be enhanced.

The immune checkpoint inhibitor contained in the cancer-cell proliferation inhibitor of the present invention is not particularly limited but include anti-PD-1 antibody, anti-PD-L1 antibody, anti-CTLA-4 antibody, anti-B7 antibody, anti-C27 antibody, anti-KIR antibody, IDO inhibitor, anti-CD137 antibody, and anti-TIM3 antibody. Anti-PD-1 antibody and anti-CTLA-4 antibody are preferable.

In the cancer-cell proliferation inhibitor of the present invention the immune checkpoint inhibitor is administered in combination with 5-(3,5-dihydroxyphenyl)-γ-valerolactone represented by the formula (I), a salt of said compound, and a conjugate of said compound. The dose of the immune checkpoint inhibitor when used in combination may be within the same range as the effective dose when used alone. The effective dose of the immune checkpoint inhibitor is not uniform. Example of the lower limit of the percentage of the immune checkpoint inhibitor to 5-(3,5-dihydroxyphenyl)-γ-valerolactone represented by the formula (I), a salt of said compound, and a conjugate of said compound is not less than 10 %, preferably not less than 20 %, more preferably not less than 50 % and the upper limit is not more than 500 %, preferably not more than 200 %, and even more preferably not more than 100 %. All the percentage here is expressed by mass %.

The cancer-cell proliferation inhibitor of the present invention can be made into the medicine or the supplement containing the immune checkpoint inhibitor and the compound represented by the formula (I) or may be made into the combination medicine consisting of different compositions. The compound represented by the formula (I) may be made into the medicine or the supplement for enhancing the effect of the immune checkpoint inhibitor.

When the present invention is made into the medicine or the supplement, the dosage form can be tablet, capsule, injection, infusion liquid, powder, suppository, granule, ointment, suspension, emulsion, syrup, cream and the like depending on the purpose and the route of the administration.

The medicine can contain the additives which are generally used for the formulation such as the binder, the excipient, the lubricant, the disintegrant or, the stabilizer, the emulsifier, the buffer. The preferable examples of the binder include guar gum and gum arabic powder. The preferable examples of the excipient include starch, trehalose, and dextrin. The preferable examples of the lubricant include stearic acid, talc, sucrose fatty acid ester, polyethylene glycol. The preferable examples of the disintegrator include starch, carboxymethylcellulose, cornstarch. The preferable examples of the stabilizer include oils and fats, propylene glycol, cyclodextrin. The preferable examples of the emulsifier include anionic surfactant, nonionic surfactant, polyvinyl alcohol. The preferable examples of the buffer include the buffer solution of phosphate, carbonate, citrate, and the like.

At least one of 5-(3,5-dihydroxyphenyl)-γ-valerolactone represented by the formula (I), a salt of said compound, and a conjugate of said compound is contained in the cancer-cell-proliferation inhibition enhancer for enhancing the effect of the immune checkpoint inhibitor of inhibiting the cancer-cell proliferation. When at least one of 5-(3,5-dihydroxyphenyl)-γ-valerolactone represented by the formula (I), a salt of said compound, and a conjugate of said compound is used as the cancer-cell-proliferation inhibition enhancer, the cancer-cell-proliferation inhibition enhancer may not contain the immune checkpoint inhibitor. Namely, when the prescribed time has passed after the immune checkpoint inhibitor is administered, the cancer-cell-proliferation inhibition enhancer may be administered. Or when the prescribed time has passed after the cancer-cell-proliferation inhibition enhancer is administered, the immune checkpoint inhibitor may be administered. The immune checkpoint inhibitor may be administered according to each prescribed usage, and typically, it is administered at intervals of a few weeks, over a period ranging from a few months up to approximately one year. The cancer-cell-proliferation inhibition enhancer is preferably administered continuously during the administration period of the immune checkpoint inhibitor and is preferably administered once in 1 to 7 days, more preferably everyday. The cancer-cell-proliferation inhibition enhancer is preferably administered continuously for from 1 week to 2 months before and after the administration period of the immune checkpoint inhibitor in the same manner. By administering in combination with the immune checkpoint inhibitor as described above the cancer-cell-proliferation inhibition enhancer work synergistically and the cancer-cell proliferation is effectively inhibited.

### Examples

The embodiments of the present invention are now described in more detail with reference to Examples as follows and is not limit to these Examples only for properly describing the embodiments of the present invention. In Manufacturing Examples and Examples described below, 5-(3,5-dihydroxyphenyl)-γ-valerolactone is also described as EGC-M5.

### <Manufacturing Example 1: Manufacture of (R)-5-(3,5-dihydroxyphenyl)-γ-valerolactone>

Eggerthella lenta strain JCM9979 was inoculated into 30 ml of GAM broth media and was incubated under the anaerobic condition at 37 °C for 48 hours to obtain the preculture solution. E.coli strain K12 and Eubacterium plautii (Flavoifractor plautii) strain ATCC29863 were incubated in 10 ml of GAM broth media under the anaerobic condition for 24 hours to obtain the preculture solutions. The preculture solutions of strain JCM9979 and E.coli strain K12 were added to 100 ml of GAM broth media containing 290 mg of (-)-epigallocatechin and incubated under the anaerobic condition at 37 °C for 48 hours. 1 ml of sample was taken from the culture solution and centrifuged at high-speed (15000 × g, 10 min.) to remove the bacteria cell. The supernatant was analyzed by LC/MS to confirm the production of (S)-1-(3,5-dihydroxyphenyl)-3-(2,4,6-trihydroxyphenyl)-propane-2-ol. The conditions of the LC/MS are described below.

### <Conditions of LC/MS>

·Column: CAPCELLPAK C18 MG (2.0i.d. × 100.0 mm, 5 um, manufactured by Shiseido Co., Ltd.)
·Flow Velocity: 0.2 ml/min.
·Temperature of Column: 40 °C
·Mobile Phase:
Solvent A: (water/acetonitrile/acetic acid = 100:2.5:0.1, volume ratio (v/v/v))
Solvent B: (water/acetonitrile/methanol/acetic acid = 35:2.5:65:0.1, volume ratio (v/v/v/v))
Gradient; 0 min.: A 100 % B 0 %, 3 min.: A 100 % B 0 %, 25 min.: A 0 % B 100 %, 25.1 min.: A 100 % B 0 %, 33 min.: A 100 % B 0 %
·Detector: UV 270 nm
·Interface: ESI
·Polarity: negative

Next, the preculture solution of strain ATCC29863 described above was added to the culture solution described above in which the production of (S)-1-(3,5-dihydroxyphenyl)-3-(2,4,6-trihydroxyphenyl)-propane-2-ol was confirmed and incubated under the anaerobic condition at 37 °C for 48 hours. After that 1 ml of sample was taken from the culture solution and centrifuged at high-speed (15000 × g, 10 min.). The supernatant obtained was analyzed by LC/MS described above to confirm the production of (R)-5-(3,5-dihydroxyphenyl)-γ-valerolactone (EGC-M5).

The culture solution in which the production of (R)-5-(3,5-dihydroxyphenyl)-γ-valerolactone was confirmed was centrifuged at high speed (10000 × g, 20 min., 10 °C) to remove the bacterial cell. Hydrochloric acid was added to the supernatant to adjust the pH to be 3.5, then the solution was extracted with 200 ml of ethyl acetate 3 times. The ethyl acetate layer was concentrated and dried by an evaporator and then provided to the preparative HPLC. The preparative HPLC condition is described below.

### <Conditions of Preparative HPLC>

·Column: CAPCELL PAK MG (20i.d. × 150 mm, 5 um, manufactured by Shiseido Co., Ltd.)
·Flow Velocity: 15 ml/min.
·Temperature of Column: 40 °C
·Mobile Phase:
Solvent A: acetonitrile:methanol:water:acetic acid (5:5:90:0.3, volume ratio (v/v/v/v))
Solvent B: acetonitrile:methanol:water:acetic acid (5:65:30:0.5, volume ratio (v/v/v/v))
Gradient; 0 min.: A 80 % B 20 %, 5 min.: A 80 % B 20 %, 20 min.: A 10 % B 90 %, 25 min.: A 1 % B 90 0,26 min.: A 80 % B 20 %, 35 min.: A 80 % B 20 %
·Detector: UV 270 nm

After fractionation by the preparative HPLC, the fraction containing the target metabolite was identified by analysis under the same conditions as the LC/MS analysis mentioned above and then concentrated and dried again by evaporator. 5 ml of water was added to the dried residue, and it was re-dissolved, then re-concentrated and dried under the reduced pressure. This process was repeated three times to fully remove the acids contained in the organic solvent. A small amount of water was added to re-dissolve the dried residue, which was then freeze-dried. 45.0 mg of (R)-5-(3,5-dihydroxyphenyl)-γ-valerolactone (EGC-M5) was obtained.

### <Manufacturing Example 2: Manufacture of (R)-5-(3-sulfoxy-5-hydroxyphenyl)-γ-valerolactone>

To 163.1 mg (0.78 mmol) of (R)-5-(3,5-dihydroxyphenyl)-γ-valerolactone obtained in Manufacturing Example 1, 10 ml of pyridine and 5 mg of sodium sulfate were added and stirred at the room temperature for 1 0 min. Next, 790.4 mg (4.68 mmol) of pyridine-sulfur trioxide complex was added and stirred at the room temperature for 1 hour. After stirring, we stopped the reaction by adding 0.2 M sodium dihydrogen phosphate buffer (pH 7.2). The concentrated residue obtained by concentrating under reduced pressure by evaporator was dissolved in 3 ml of water and subjected to the preparative HPLC. The condition of the preparative HPLC was described below.

### <Conditions of Preparative HPLC>

·Column: CAPCELLPAK MG (20i.d. × 150 mm, 5 um, manufactured by Shiseido Co., Ltd.)
·Flow Velocity: 19.0 ml/min.
·Temperature of Column: 40 °C
·Mobile Phase:
Solvent A: sodium perchlorate/water (122.4:500, weight/volume ratio (w/v))
Solvent B: sodium perchlorate/acetonitrile (122.4:500, weight/volume ratio (w/v))
Gradient; 0 min.: A 100 % B 0 %, 3 min.: A 100 % B 0 %, 15 min.: A 0 % B 100 %, 16 min.: A 100 % B 0 0,20 min.: A 100 % B 0 %
·Detector: UV 280 nm

Acetonitrile was removed by concentrating the fraction under reduced pressure by evaporator to obtain the roughly purified (R)-5-(3-sulfoxy-5-hydroxyphenyl)-γ-valerolactone. Each roughly purified product obtained was introduced into the column obtained by additionally filling SUPELCO DISCOVERY DSC-18 column with ODS (Chromatorex 15-30 µm, 10 cm³). After washing with 160 ml of water, the roughly purified product was eluted with 175 ml of acetonitrile. The acetonitrile elution fractions obtained were concentrated under reduced pressure by evaporator and freeze-dried respectively to obtain 12.5 mg (0.04 mmol, yield 5.2 %) of the purified (R)-5-(3-sulfoxy-5-hydroxyphenyl)-γ-valerolactone in white powder form.

The chemical shift value described below was obtained by 1H-NMR analysis of the purified product obtained and it was confirmed that the purified product obtained was the desired compound.

(R)-5-(3-sulfoxy-5-hydroxyphenyl)-γ-valerolactone: 1H-NMR (400MHz, deuterated methanol): 56.24 (2H, s), 6.19 (1H, s), 4.79 (1H, m), 2.91 (2H, d, j=6.8Hz), 2.55 (1H, m),2.46(1H, m), 2.33 (1H, m), 2.00 (1H, m)

### <Manufacturing Example 3: Manufacture of (R)-5-[3-(β-D-gulcopyranuronosyloxy-5-hydroxy) phenyl]-γ-valerolactone>

Six 8- weeks-old Wistar series rats (male, purchased from Charles River Laboratories Japan, Inc.) preliminarily reared on the purified feed for a week were fasted from the day before the test and used for the test. The sample for administering was the solution obtained by dissolving (-)-epigallocatechin in the physiological saline solution. 20 mg/1.7 ml of the sample was forcibly administered to each rat into the stomach. The administration was repeated for 4 days, and urine was collected from 6 to 24 hours after each administration.

250 ml of the urine collected was put together and concentrated by evaporator under the reduced pressure to reduce the volume of the urine to about 45 ml. Next, 225 ml of methanol was added to the concentrate obtained and stirred and then centrifuged at high speed (10000 × g, for 10 min., 4 °C) to remove the precipitated protein. The supernatant obtained was concentrated by evaporation under reduced pressure to reduce the volume to approximately 45 ml. After water was added to the concentrate obtained to increase the volume to 450 ml, pH was adjusted to be 3. 0 by adding acetic acid. After the adjusted urine solution was passed through OasisHLB cartridge (manufactured by Waters Corporation, 35 cc) whose pH was adjusted to be 3.0 with 0.1 M phosphate -citrate acid buffer, the cartridge was washed with 175 ml of water and 175 ml of 20 % methanol aqueous solution in this order. Then the fraction containing (R)-5-[3-(β-D-gulcopyranuronosyloxy-5-hydroxy) phenyl]-γ-valerolactone was eluted with 175 ml 40 % methanol aqueous solution. The 40 % methanol elution fraction obtained was concentrated by evaporator under reduced pressure and 5 ml of water was added to the concentrated residue and the solution was subjected to the preparative HPLC. The condition of the preparative HPLC is described below.

### <Conditions of Preparative HPLC>

·Column: Mightysil RP18 GP (20i.d. × 250 mm, 5 um, manufactured by KANTOU CHEMICAL CO., INC.)
·Flow Velocity: 15 ml/min.
·Mobile Phase:
Solvent A: methanol/water/acetic acid (5/95/0.2, volume ratio (v/v/v))
Solvent B: methanol/water/acetic acid (60/40/0.2, volume ratio (v/v/v))
Gradient; 0 min.: A 80 % B 20 %, 5 min.: A 80 % B 20 %, 15 min.: A 0 % B 100 %, 18 min.: A 0 % B 100 %, 18.1 min.: A 80 % B 20 %, 25 min.: A 80 % B 20 %
·Detector: UV 270 nm

After fractionation by the preparative HPLC, the obtained fractionated solution was subjected to LC/MS analysis (under the same conditions as in Manufacturing Example 1) to confirm that (R)-5-[3-(β-D-gulcopyranuronosyloxy-5-hydroxy) phenyl]-γ-valerolactone was contained.

Additionally, the fraction containing (R)-5-[3-(β-D-gulcopyranuronosyloxy-5-hydroxy) phenyl]-γ-valerolactone was subjected to recycling HPLC to purify. The condition of the recycling HPLC was described below.

### <Conditions of Recycling HPLC>

·Column: Mightysil RP18 GP (20i.d. × 250 mm, 5 um, manufactured by KANTOU CHEMICAL CO., INC.)
·Flow Velocity: 15 ml/min.
·Mobile Phase: acetonitrile/water/acetic acid (15/85/0.2, volume ratio (v/v/v))
·Detector: UV 270 nm

After purification by recycling HPLC, the fraction was confirmed to contain (R)-5-[3-(β-D-gulcopyranuronosyloxy-5-hydroxy) phenyl]-γ-valerolactone by LC/MS analysis described above. The fraction containing (R)-5-[3-(β-D-gulcopyranuronosyloxy-5-hydroxy) phenyl]-γ-valerolactone was concentrated and dried by evaporator. 5 ml of water was added to the dried residue, and it was re-dissolved, then re-concentrated and dried under reduced pressure. This process was repeatedthree times to fully remove the acids contained in the organic solvent. Finally, 2 ml of water was added to re-dissolve the dried residue, which was then freeze-dried. 28.0 mg of (R)-5-[3-(β-D-gulcopyranuronosyloxy-5-hydroxy) phenyl]-γ-valerolactone was obtained.

### <Example 1: Effect of Combined Administration of EGC-M5 and Anti-CTLA-4 Antibody on Colonic Cancer Cell Proliferation>

### (a) Rearing Procedure and Administering Procedure

After the preparatory rearing, MC38 mouse colon cancer cell line was transplanted subcutaneously into 6-weeks-old male C57BL/6J mice at an amount of 5.0 × 10⁵ cells/mouse. Each group had 8 mice. Anti-CTLA-4 antibody (manufactured by BioX Cell) at a dose of 2.5 mg/kg b.w. was administered intraperitoneally to the mice in the anti-CTLA-4 antibody alone group every 7 days from the 4th day after transplantation. The mice in the combination administration group were intraperitoneally injected with anti-CTLA-4 antibody at a dose of 2.5 mg/kg b.w. every 7 days and with EGC-M5 at a dose of 10 mg/kg b.w. daily from the 4th day after transplantation. The test substances were dissolved in the PBS buffer with 10 % DMSO. The Control group, in which the mice were not treated with anti-CTLA-4 antibody, received the isotype control antibody (Isotype control antibody, manufactured by BioX Cell). The tumor volume was measured with the vernier calipers and the growth of the tumor was evaluated.

### (b) Results of Test

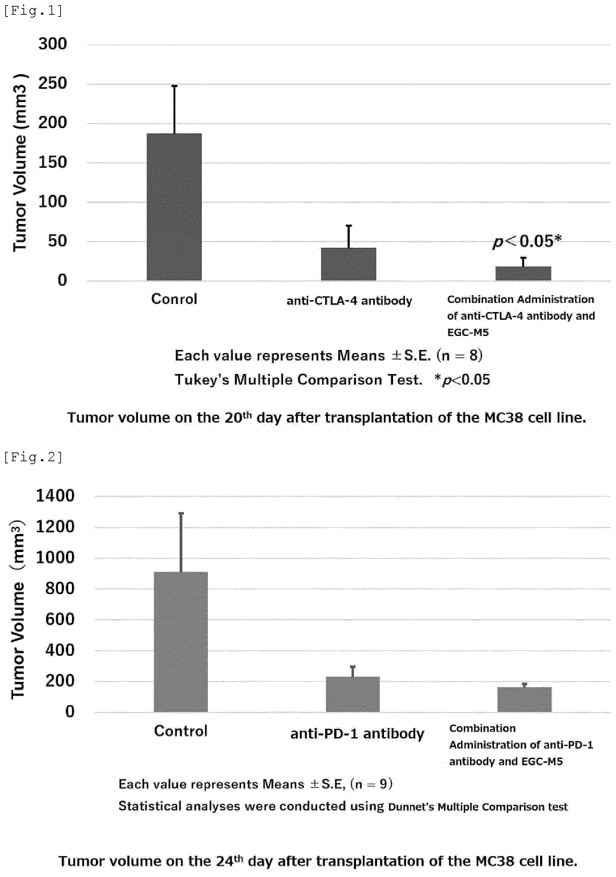

As shown in Fig.1 there was a significant difference in tumor volume values between the control group and the combination administration group on the 20th day after transplantation. On the other hands, no significant change in tumor volume was observed in the group treated solely with the anti-CTLA-4 antibody. Additionally, in the combination administration group, two mice were confirmed to be in remission, with all tumor cells eradicated at the time of euthanasia. These results indicate that the combined administration of EGC-M5 and anti-CTLA-4 antibody enhances the anti-tumor effect.

### (c) Statistical Processing Method

The results of the tumor volume were represented as mean (Mean) and the standard error (S.E.), and the significance of differences was assessed using Tukey's Multiple Comparison test. The level of significance was set to *P<0.05.

### <Example 2: Effect of Combination Administration of EGC-M5 and Anti-PD-1 Antibody against Colonic Cancer-Cell Proliferation>

### (a) Rearing Procedure and Administering Procedure

After preparatory rearing, MC38 mouse colon cancer cell line was transplanted subcutaneously into 6-weeks-old male C57BL/6J mice at an amount of 5.0 × 10⁵ cells/mouse. Each group had 10 mice. Mice in the group administered with anti-PD-1 antibody alone were intraperitoneally injected with anti-PD-1 antibody (manufactured by BioX Cell) at a dose of 5 mg/kg b.w., four times on days 4, 7, 11, and 14 after transplantation. The mice in the combination administration group were intraperitoneally injected with anti-PD-1 antibody at a dose of 5 mg/kg b.w., four times on days 4, 7, 11, and 14 after transplantation and with EGC-M5 at a dose of 10 mg/kg b.w. daily starting on day 4 after transplantation. The test substances were dissolved in the PBS buffer with 10 % DMSO. Isotype control antibody (Isotype control antibody manufactured by BioX Cell) was administered to the mice in the Control group in which the mice were not dosed with anti-PD-1 antibody. The tumor volume was measured with the vernier calipers every 2 days and the growth of the tumor was evaluated. Subsequently, when the tumor volume reached the end point (3000 mm³), the mice were euthanized and the survival rate was assessed.

### (b) Results of Test

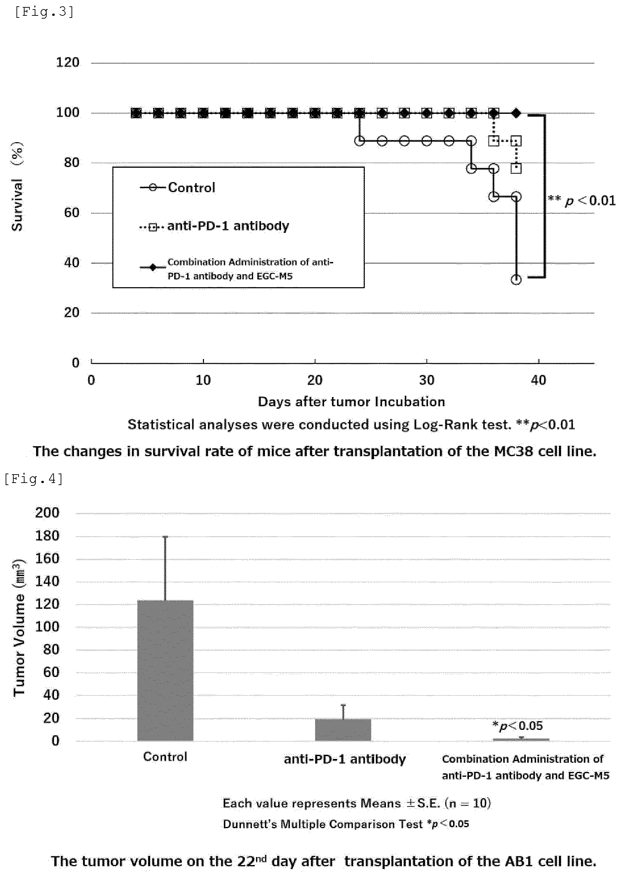

As shown in Fig.2 and 3, the combination treatment of EGC-M5 and anti-PD-1 antibody suppressed tumor growth and increased survival rate more than the anti-PD-1 antibody treatment alone.

The measurement of the tumor volume on the 24th day post-transplantation revealed that the tumor growth was significantly restrained by the combined treatment of EGC-M5 and anti-PD-1 antibody. As a result of the evaluation of the survival rate on the 38^{th} day after tumor transplantation, the survival rate increased significantly in the combination administration group. From these results, it was clear that EGC-M5 can amplify the anti-tumor activity of the anti-PD-1 antibody.

### (c) Statistical Processing Method

The results of the tumor volume were represented by the mean (Mean) and the standard error (S.E.) and the significant difference from the control group was assessed by Dunnet's Multiple Comparison test. Regarding the survival rate results, the significant difference from the control group was evaluated with the Log-Rank test. The level of significance was set to *P<0.01.

### <Example 3: Effect of Combination Administration of EGC-M5 and Anti-PD-1 Antibody against Malignant Pleural Mesothelioma-Cell Proliferation>

### (a) Rearing Procedure and Administering Procedure

After the preparatory rearing, AB1 malignant pleural mesothelioma cell line was transplanted subcutaneously into the 6-weeks-old male C57BL/6J mice at an amount of 1.0×10⁷ cells/mouse. Each group had 9 mice. The mice in the anti-PD-1 antibody alone treatment group were intraperitoneally injected with anti-PD-1 antibody (manufactured by BioX Cell) at a dose of 5 mg/kg b.w. four times on days 4, 7, 10, and 14 after transplantation. The mice in the combination treatment group were intraperitoneally injected with anti-PD-1 antibody (manufactured by BioX Cell) at a dose of 5 mg/kg b.w. intraperitoneally four times on days 4, 7, 10, and 14 after transplantation. In addition, EGC-M5 started to be given daily, at a dose of 10 mg/kg b.w. from day 4 after transplantation. The test substances were dissolved in the PBS buffer with 10 % DMSO. The mice in Control group in which the mice were not administered with anti-PD-1 antibody were administered with isotype control antibody (Isotype control antibody manufactured by BioX Cell). The volume of the tumor was measured with the vernier calipers every 3 days and the growth of the tumor was evaluated.

### (b) Results of Test

As shown in Fig.4, the tumor growth was significantly inhibited by the combined administration of EGC-M5 and anti-PD-1 antibody compared to Control group. At 22 days after transplantation, no mice in Control group, 4 mice in the anti-PD-1 antibody alone administration group, and 6 mice in the combination administration group were confirmed to be in remission. The above results confirmed that EGC-M5 enhanced the anti-tumor effect of anti-PD-1 antibody when used together with anti-PD-1 antibody.

### (c) Statistical Processing Method

The results of the tumor volume were represented as mean (Mean) and the standard error (S.E.), and the significance of differences from the control group was assessed using Dunnet's Multiple Comparison test. The level of significance was set to *P<0.05.

### <Comparative Example: Effect of Administration of EGC-M5 Alone against Colonic Cancer Cell Proliferation>

### (a) Rearing Procedure and Administering Procedure

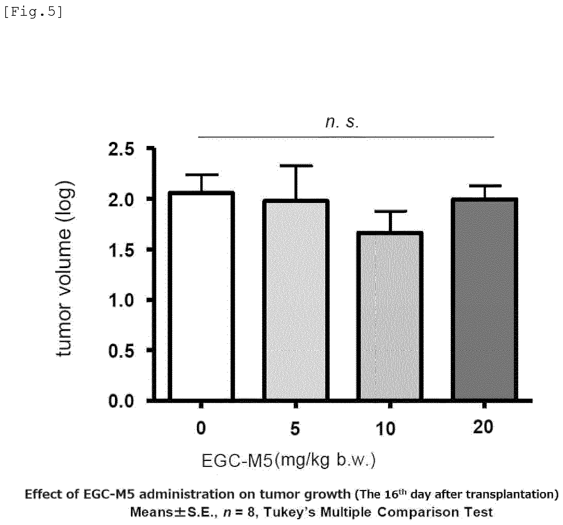

After preparatory rearing, MC38 mouse colon cancer cell line was transplanted subcutaneously into the 6-weeks-old male C57BL/6 mice at an amount of 5.0 × 10⁵ cells/mouse. Each group had 8 mice. EGC-M5 was dissolved in the PBS buffer containing 10 % DMSO. 200 µL of EGC-M5 solution at a dose of 5 mg, 10 mg, 20 mg, and 30 mg/kg b.w. was intraperitoneally administered to the mice daily starting on day 4 after transplantation. The tumor volume was measured with the vernier calipers, and the growth of the tumor was evaluated with time. The means of the tumor volume (natural logarithm) on the 16th day after transplantation was shown in Fig. 5.

### (b) Results of Test

Regarding to the mean (Mean) and the standard error (S.E.) of the tumor volume (natural logarithm) 16 days after transplantation, the significant difference between the control group (Control group) and the EGC-M5 administration group was assessed by Tukey's Multiple Comparison test. As a result, no significant differences were observed in the tumor volume values between the two groups.

### Industrial Applicability

According to this invention, the specific compound and its conjugates, reported as catechin metabolites, can enhance the effects of immune checkpoint inhibitors. This invention can provide a cancer cell proliferation inhibitor comprising immune checkpoint inhibitors and the compound represented by the formula (I). Furthermore, the specific compound and its conjugates can serve as an enhancer of the anticancer effect of the immune checkpoint inhibitor. This invention provides safe and consumable food supplements and drugs whose active ingredients are derived from food.

## Claims

1. A cancer-cell proliferation inhibitor comprising an immune checkpoint inhibitor and at least one of 5-(3,5-dihydroxyphenyl)-γ-valerolactone, which is represented by a following formula (I), a salt of said compound, and a conjugate of said compound.

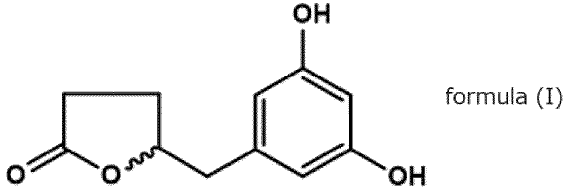

2. The cancer-cell proliferation inhibitor according to Claim 1, wherein the immune checkpoint inhibitor is one or more selected from a group consisting of anti-PD-1 antibody, anti-PD-L1 antibody, anti-CTLA-4 antibody, anti-B7 antibody, anti-C27 antibody, anti-KIR antibody, IDO inhibitor, anti-CD137 antibody, and anti-TIM3 antibody.

3. The cancer-cell proliferation inhibitor according to Claim 2, wherein the immune checkpoint inhibitor is anti-PD-1 antibody, or anti-CTLA-4 antibody.

4. A medicine for inhibiting cancer-cell proliferation containing the cancer-cell proliferation inhibitor according to any one of Claims 1 to 3.

5. A supplement for inhibiting cancer-cell proliferation containing the cancer-cell proliferation inhibitor according to any one of Claims 1 to 3.

6. A method for enhancing effect of an immune checkpoint inhibitor, wherein anticancer effect of the immune checkpoint inhibitor is enhanced by using the immune checkpoint inhibitor and a cancer-cell proliferation inhibitor comprising at least one of 5-(3,5-dihydroxyphenyl)-γ-valerolactone, which is represented by a following formula (I), a salt of said compound, and a conjugate of said compound as an active ingredient together.

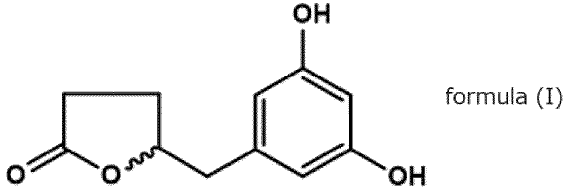

7. A cancer-cell-proliferation inhibition effect enhancer for enhancing cancer-cell-proliferation inhibiting effect of an immune checkpoint inhibitor, comprising at least one of 5-(3,5-dihydroxyphenyl)-γ-valerolactone, which is represented by a following formula (I), a salt of said compound, and a conjugate of said compound.
